# EUROPEAN PATENT APPLICATION

(11) **EP 4 744 722 A1**
(43) Date of publication of application: **20.05.2026**
(21) Application number: 24858627.3
(22) Date of filing: 28.08.2024
(51) Int. Cl.: A61N 1/375, A61B 5/25, A61N 1/36, A61N 1/04

(54) **ELECTRODE WIRE, STIMULATOR SYSTEM, AND COMPUTER-READABLE STORAGE MEDIUM**

(30) Priority: 30.08.2023 CN 202311105287; 30.08.2023 CN 202322341916 U
(71) Applicant: Sceneray Co., Ltd, Suzhou, Jiangsu 215000 (CN)
(72) Inventor: ZHI, Menghui, Suzhou, Jiangsu 215000 (CN); ZHU, Weiran, Suzhou, Jiangsu 215000 (CN)
(74) Representative: Cabinet Beaumont
(86) International application number: PCT/CN2024/115204
(87) International publication number: WO 2025/045094

(57) **Abstract**

Provided are an electrode lead, a stimulator system, and a computer-readable storage medium. The electrode lead includes a catheter, at least one electrode contact, and a signal processing chip. Each electrode contact is attached to an outer wall of the catheter and is configured to perform at least one of the following: acquiring a bioelectrical signal of an object, or delivering electrical stimulation to internal tissue of the object based on a stimulation pulse signal. The signal processing chip is disposed within the catheter and is electrically connected to the at least one electrode contact.

## Description

This application claims priority to Chinese Patent Application No. 202311105287.X and Chinese Patent Application No. 202322341916.0 filed with the China National Intellectual Property Administration (CNIPA) on Aug. 30, 2023, the disclosures of which are incorporated herein by reference in their entireties.

### TECHNICAL FIELD

The present application relates to the technical field of medical devices, for example, an electrode lead, a stimulator system, and a computer-readable storage medium.

### BACKGROUND

An electrode contact serves as a carrier for electrical stimulation treatment and bioelectrical signal acquisition, and is electrically connected to a pulse generator through a corresponding cable (an electrode lead and an extension lead). In general, the amplitude of a bioelectrical signal is on the order of µV, and the amplitude of a stimulation pulse signal is on the order of V, differing by six orders of magnitude. Even with a shielding layer added to the cable, the bioelectrical signal (an analog signal) is still highly susceptible to interference from the external environment and from the stimulation pulse signal within the cable, which poses a challenge for the parsing of the bioelectrical signal and the subsequent closed-loop control of the bioelectrical signal.

### SUMMARY

The present application provides an electrode lead, a stimulator system, and a computer-readable storage medium, which can reduce the external interference and the interference of a stimulation pulse signal on a bioelectrical signal by integrating a signal processing chip into the electrode lead.

In a first aspect, the present application provides an electrode lead. The electrode lead includes a catheter, at least one electrode contact, and a signal processing chip.

Each electrode contact of the at least one electrode contact is attached to an outer wall of the catheter, and each electrode contact is configured to perform at least one of the following: acquiring a bioelectrical signal of an object, or delivering electrical stimulation to internal tissue of the object based on a stimulation pulse signal.

The signal processing chip is disposed within the catheter and is electrically connected to the at least one electrode contact.

In some optional embodiments, the signal processing chip is integrated with a signal parsing circuit and a signal acquisition circuit, where the signal parsing circuit is configured to parse a stimulation control instruction sent by a pulse generator and convert the stimulation control instruction into the stimulation pulse signal, and the signal acquisition circuit is configured to convert bioelectrical signals acquired by the at least one electrode contact into to-be-detected digital signals and output the to-be-detected digital signals.

In some optional embodiments, multiple groups of electrode contacts are arranged along a length direction of the catheter, and electrode contacts in each group of the multiple groups of electrode contacts are uniformly distributed in a circumferential direction of the catheter.

In some optional embodiments, for the electrode contacts in each group, when any electrode contact delivers the electrical stimulation to the internal tissue of the object based on the stimulation pulse signal, part or all of other electrode contacts in the same group acquire bioelectrical signals of the object.

In some optional embodiments, the electrode lead is provided with two to eight groups of electrode contacts among the multiple groups of electrode contacts.

In some optional embodiments, the electrode lead is provided with four groups of electrode contacts among the multiple groups of electrode contacts.

In some optional embodiments, each group includes one to nine electrode contacts.

In some optional embodiments, each group includes three electrode contacts.

In some optional embodiments, the catheter is a rubber tube, the signal processing chip includes at least one bare die, the signal processing chip is electrically connected to each electrode contact via a metal lead, and the at least one bare die is electrically connected to each other via metal leads.

In some optional embodiments, the signal processing chip includes one to ten bare dies.

In some optional embodiments, the signal processing chip includes three bare dies.

In some optional embodiments, the three bare dies form a triangular prism structure.

In some optional embodiments, an adhesive is filled between the signal processing chip and an inner wall of the catheter to secure the signal processing chip within the catheter.

In some optional embodiments, the signal acquisition circuit includes an amplification module, a band-pass filtering module, and an analog-to-digital conversion module that are connected in series.

The amplification module is configured to amplify the bioelectrical signals to obtain first intermediate signals.

The band-pass filtering module is configured to perform band-pass filtering on the first intermediate signals to obtain second intermediate signals.

The analog-to-digital conversion module is configured to perform analog-to-digital conversion on the second intermediate signals to obtain the to-be-detected digital signals.

In some optional embodiments, the internal tissue of the object includes subthalamic nucleus.

In some optional embodiments, the bioelectrical signal includes a local field potential signal.

In a second aspect, the present application includes a stimulator system. The stimulator system includes a pulse generator and the electrode lead according to any one of the preceding solutions, where the pulse generator is electrically connected to the signal processing chip in the electrode lead.

In some optional embodiments, the pulse generator includes a memory and at least one processor, the memory stores a computer program, and when executing the computer program, the at least one processor is configured to perform the following steps.

To-be-detected digital signals output by the signal processing chip are received.

A stimulation control instruction is generated according to the to-be-detected digital signals, and the stimulation control instruction is output to the signal processing chip.

In some optional embodiments, when executing the computer program, the at least one processor is further configured to perform the following steps.

Disease information of the object is acquired.

A first duration and a second duration within a signal acquisition period are set according to the disease information and the to-be-detected digital signals, where multiple electrode contacts are provided.

During the first duration, at least one electrode contact of the electrode contacts transmits the stimulation pulse signal to the internal tissue of the object, and at least one of remaining electrode contacts of the electrode contacts acquires bioelectrical signals of the object.

During the second duration, the at least one electrode contact of the electrode contacts does not transmit the stimulation pulse signal, and at least one of the remaining electrode contacts of the electrode contacts acquires bioelectrical signals of the object.

In a third aspect, the present application provides a computer-readable storage medium storing a computer program that, when executed by at least one processor, is configured to fulfill functions of the stimulator system according to any one of the preceding solutions.

In a fourth aspect, the present application further provides a computer program product including a computer program that, when executed by at least one processor, is configured to fulfill functions of the stimulator system according to any one of the preceding solutions.

### BRIEF DESCRIPTION OF DRAWINGS

The present application is described below in connection with drawings and embodiments.
FIG. 1 is a diagram illustrating the structure of an electrode lead according to an embodiment of the present application.
FIG. 2 is another diagram illustrating the structure of an electrode lead according to an embodiment of the present application.
FIG. 3 is a diagram illustrating the principle of signal transmission of a stimulator system according to an embodiment of the present application.
FIG. 4 is a block diagram of a stimulator system according to an embodiment of the present application.
FIG. 5 is a diagram illustrating the structure of a program product according to an embodiment of the present application.
FIG. 6 is a block diagram of a signal processing chip according to an embodiment of the present application.

### DETAILED DESCRIPTION

Technical solutions in the present application are described below in connection with the drawings and embodiments of the present specification. If there is no conflict, multiple embodiments or technical features described below may be combined in any manner to form new embodiments.

In the embodiments of the present application, "at least one" refers to one or more, and "multiple" refers to two or more. "And/or" is used for describing an association between associated objects and indicates three relations. For example, "A and/or B" may indicate the presence of A alone, the presence of both A and B, and the presence of B alone, where A and B may be singular or plural. The character "/" generally indicates an "or" relation between the associated objects. "At least one of the following" or a similar expression thereof refers to any combination of items, including any combination of singular items or plural items. For example, at least one of a, b, or c may indicate a, b, c, "a and b", "a and c", "b and c", or "a and b and c", where a, b, and c may be singular or plural. It is to be noted that "at least one" may also be interpreted as "one or more."

In the embodiments of the present application, the term "exemplarily" or "for example" are used to indicate illustration, example, or explanation. Any embodiment or design solution described as "exemplarily" or "for example" in the embodiments of the present application should not be construed as being preferred or superior to other embodiments or design solutions. The use of the term such as "exemplarily" or "for example" is intended to present relevant concepts in a concrete manner.

One application field of the present application (that is, implantable nerve stimulators) is first briefly described.

Implantable medical systems typically include an implantable nerve stimulation system, an implantable cardiac stimulation system (known as a cardiac pacemaker), an implantable drug delivery system, and a lead adapter system. Examples of the implantable nerve stimulation system include a deep brain stimulation (DBS) system, an implantable cortical nerve stimulation (CNS) system, an implantable spinal cord stimulation (SCS) system, an implantable sacral nerve stimulation (SNS) system, and an implantable vagus nerve stimulation (VNS) system.

The implantable nerve stimulation system includes an implantable target object, such as a stimulator implanted in a patient (that is, an implantable nerve stimulator, which is a nerve stimulation apparatus), and a program-controlled device located outside the body of the patient. That is, the stimulator is an implant, or an implant includes the stimulator. Relevant neuromodulation technologies mainly involve implanting an electrode (such as an electrode lead) into a specific site (that is, a target) of biological tissue through stereotactic surgery. A discharge pulse is delivered to the target via the implanted electrode to modulate the electrical activity and function of a corresponding nerve structure and network, thereby improving symptoms and alleviating pain. The stimulator may include an implantable pulse generator (IPG), an extension lead, and an electrode lead. The IPG is implanted inside the patient and, in response to a program-controlled command sent by the program-controlled device, provides controllable electrical stimulation energy to internal tissue through a sealed battery and a circuit. The IPG delivers one or more channels of controllable and specific electrical stimulation to a specific region of the internal tissue via the extension lead and the electrode lead. The extension lead is used in combination with the IPG as a medium for transmitting an electrical stimulation signal, transmitting an electrical stimulation signal generated by the IPG to the electrode lead. The electrode lead delivers the electrical stimulation to the specific region of the internal tissue through multiple electrode contacts. The stimulator is provided with one or more electrode leads on one side or both sides, and each electrode lead is provided with multiple electrode contacts. The electrode contacts may be arranged evenly or unevenly in a circumferential direction of each electrode lead. As an example, the electrode contacts may be arranged in the circumferential direction of each electrode lead in an array of four rows and three columns (a total of 12 electrode contacts). The electrode contacts may include stimulation electrode contacts and/or acquisition electrode contacts. The electrode contacts may be plate-shaped, ring-shaped, or dot-shaped.

In some other embodiments, the stimulator only includes the pulse generator and the electrode lead. The pulse generator is embedded onto the skull of the patient, and the electrode lead is implanted intracranially. In this case, the pulse generator is directly connected to the electrode lead without requiring an extension lead.

As an example, the deep brain stimulation (DBS) system includes an IPG, an extension lead, and an electrode lead, with the IPG connected to the electrode lead via the extension lead. The IPG is implanted into the body of the patient, for example, into the chest of the patient or another internal site of the patient.

As another example, the DBS system includes an IPG and an electrode lead, with the IPG directly connected to the electrode lead. The IPG is implanted into the head of the patient. For example, a groove is formed in the skull of the patient, and the IPG is installed within the groove. In this case, the IPG may either be flush with or partially protrude from the outer surface of the skull.

In some embodiments, the internal tissue being stimulated may be the brain tissue of the patient, and a stimulation site may be a specific site of the brain tissue. The stimulation site generally varies depending on the type of disease the patient has, and the number of stimulation contacts (electrode contacts) used (single-source or multi-source), the application of one or more (single-channel or multi-channel) specific electrical stimulation signals, and stimulation parameter data also vary. The applicable types of disease in the embodiments of the present application are not limited and may be types of disease applicable to deep brain stimulation (DBS), spinal cord stimulation (SCS), pelvic stimulation, gastric stimulation, peripheral nerve stimulation, and functional electrical stimulation. The DBS may be used to treat or manage types of disease including, but not limited to: spastic disorders (such as epilepsy), pain, migraine, mental disorders (such as major depressive disorder (MDD)), bipolar disorder, anxiety, post-traumatic stress disorder (PTSD), dysthymia, obsessive-compulsive disorder (OCD), behavioral disorders, mood disorders, memory disorders, psychological state disorders, movement disorders (such as essential tremor or Parkinson's disease), Huntington's disease, Alzheimer's disease, drug addiction, autism, or other neurological or mental disorders and impairments.

In the embodiments of the present application, when a program-controlled connection is established between the program-controlled device and the stimulator, the program-controlled device may be used to adjust stimulation parameters of the stimulator (or stimulation parameters of the pulse generator, with different stimulation parameters corresponding to different electrical stimulation signals). The stimulator may also sense the electrophysiological activity of the patient to acquire a bioelectrical signal, and the acquired bioelectrical signal may be further used to continue adjusting the stimulation parameters of the stimulator.

Stimulation parameters of an electrical stimulation signal may include one or more of: a frequency (for example, the number of electrical stimulation pulse signals per unit time of 1s, in Hz), a pulse width (the duration of each pulse, in µs), an amplitude (generally expressed as a voltage, such as the intensity of each pulse, in V), a time sequence (which may be continuous or triggered), a stimulation mode (including one or more of a current mode, a voltage mode, a timed stimulation mode, and a cyclic stimulation mode), doctor-controlled upper and lower limits (the range adjustable by a doctor), and patient-controlled upper and lower limits (the range adjustable by a patient). In practical applications, the multiple stimulation parameters of the stimulator may be adjusted in either the current mode or the voltage mode.

The program-controlled device may be a user program-controller, and a user may be either a doctor or a patient, that is, a doctor program-controller or a patient program-controller. The doctor program-controller may be, for example, an intelligent terminal device equipped with program-control software, such as a tablet computer, a laptop computer, a desktop computer, or a mobile phone. The patient program-controller may be, for example, an intelligent terminal device equipped with program-control software, such as a tablet computer, a laptop computer, a desktop computer, or a mobile phone, and may also be another electronic device with the program-control function (for example, a charger or a data acquisition device that has the program-control function).

Data interaction between the doctor program-controller and the stimulator is not limited in the present application. When the doctor performs remote program control, the doctor program-controller may perform the data interaction with the stimulator through a server and the patient program-controller. When the doctor performs program control offline with the patient face-to-face, the doctor program-controller may perform the data interaction with the stimulator through the patient program-controller, and may also perform the data interaction with the stimulator directly.

The patient program-controller may include a host that communicates with the server and a sub-host that communicates with the stimulator, with a communicable connection between the host and the sub-host. The doctor program-controller may perform data interaction with the server via 3rd-generation (3G)/4G/5G mobile communication networks. The server may perform data interaction with the host via the 3G/4G/5G networks. The host may perform data interaction with the sub-host via the Bluetooth protocol/the Wireless Fidelity (Wi-Fi) protocol/the Universal Serial Bus (USB) protocol. The sub-host may perform data interaction with the stimulator via an operating band from 401 MHz to 406 MHz/an operating band from 2.4 GHz to 2.48 GHz. The doctor program-controller may perform data interaction directly with the stimulator via the operating band from 401 MHz to 406 MHz/the operating band from 2.4 GHz to 2.48 GHz.

For real-time closed-loop stimulation, the electrode contacts not only provide an electrical stimulation function but also need to perform bioelectrical signal acquisition.

The present application provides an electrode lead, a stimulator system, and a computer-readable storage medium so that the integration of a signal processing chip into the electrode lead can reduce the external interference and the interference of a stimulation pulse signal on a bioelectrical signal.

### Embodiment of the electrode lead

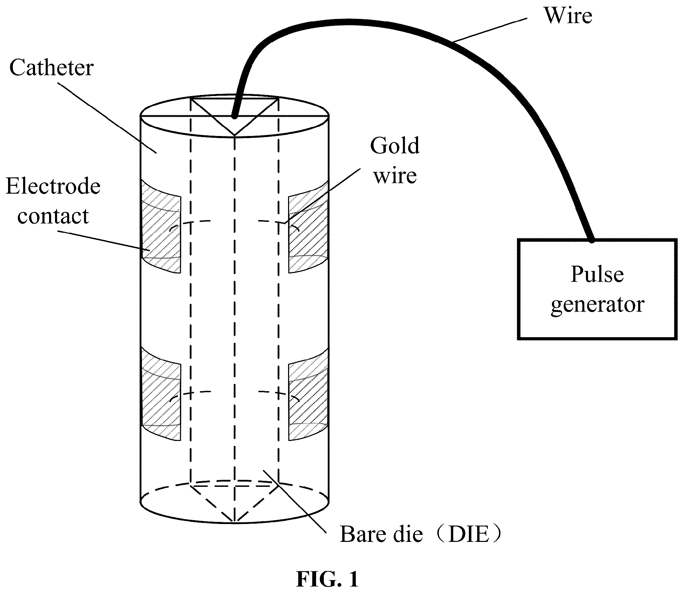

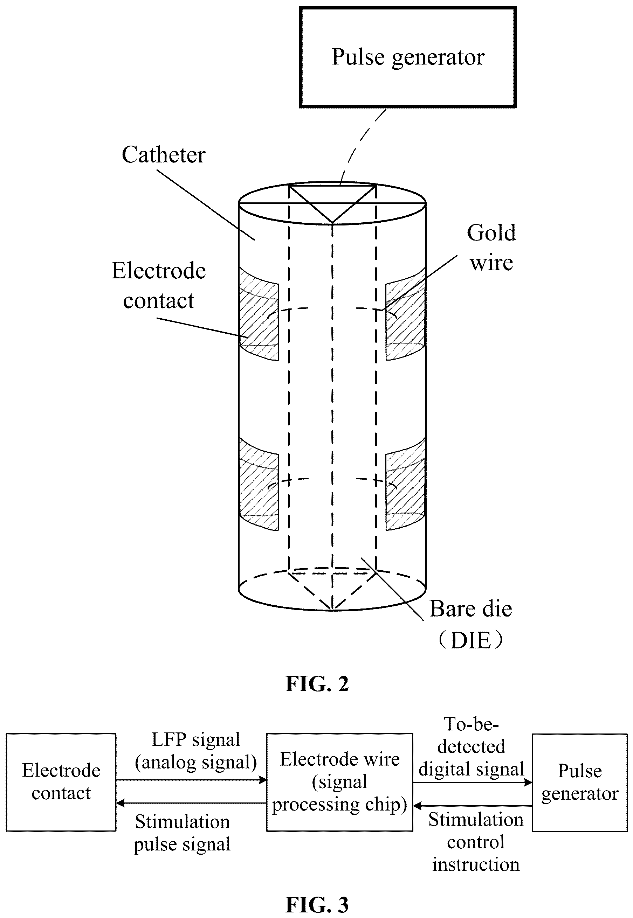

Referring to FIGS. 1 and 2, FIG. 1 is a diagram illustrating the structure of an electrode lead according to an embodiment of the present application, and FIG. 2 is another diagram illustrating the structure of an electrode lead according to an embodiment of the present application.

The electrode lead includes a catheter, at least one electrode contact, and a signal processing chip.

Each electrode contact is attached to an outer wall of the catheter, and each electrode contact is configured to perform at least one of the following: acquiring a bioelectrical signal of a patient, or delivering electrical stimulation to internal tissue of the patient based on a stimulation pulse signal.

The signal processing chip is disposed within the catheter and is electrically connected to the at least one electrode contact.

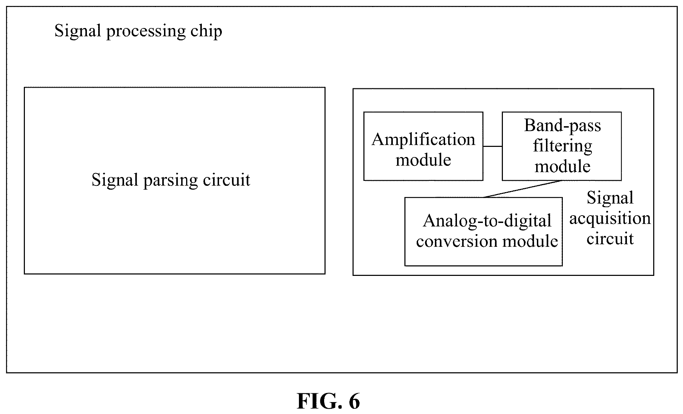

In some embodiments, as shown in FIG. 6, the signal processing chip is integrated with a signal parsing circuit and a signal acquisition circuit. The signal parsing circuit is configured to parse a stimulation control instruction sent by a pulse generator and convert the stimulation control instruction into the stimulation pulse signal, and the signal acquisition circuit is configured to convert bioelectrical signals acquired by the at least one electrode contact into to-be-detected digital signals and output the to-be-detected digital signals.

Types of the bioelectrical signals are not limited in the embodiments of the present application. The bioelectrical signals may include a neuronal signal and/or a local field potential signal.

The neuronal signal is an electrical signal generated by neuronal cells and is used for information transmission between neurons. The neuronal signal typically exists in the form of a pulse, referred to as an action potential. The acquisition and analysis of the neuronal signal can provide information about the neuronal activity, thereby helping to understand the function of a nervous system and an abnormality of the nervous system. The local field potential (LFP) signal refers to a potential change generated by the electrical activity of surrounding neurons. The acquisition of the local field potential signal can provide information about the neural network activity and the acquireive behavior of a neuronal population. The analysis of the local field potential signal can help understand the synchrony of the neuronal population, a modulation mechanism of the neuronal population, as well as the electrical activity associated with a specific function of the neuronal population. By acquiring and analyzing neuronal signals and/or local field potential signals, important information about neural system function and disease states can be obtained, which facilitates the diagnosis and treatment of neurological disorders and enables personalized closed-loop deep brain stimulation therapy.

Types of patients applicable to the electrode lead are not limited in the present application and include, for example, a patient with Parkinson's disease and a patient with obsessive-compulsive disorder. For ease of understanding, Parkinson's disease is used as an example below. Parkinson's disease is a neurodegenerative disorder whose primary symptoms include motor impairments such as tremor, bradykinesia, and muscular rigidity. The bioelectrical signals acquired may be, for example, local field potential (LFP) signals. Parkinson's disease is typically characterized by the power spectral density of a β-band (from 13 Hz to 35 Hz) of each LFP signal being higher or lower than a preset range, that is, an abnormal power spectral density. In other words, determining whether the power spectral density is abnormal can serve as an important basis for closed-loop feedback in the patient with Parkinson's disease, and can be used to adjust parameters of the electrical stimulation of the patient to improve symptoms of the patient with Parkinson's disease.

As an example, the electrode lead includes the catheter, the at least one electrode contact, and the signal processing chip. The catheter is a flexible and insulated tubular structure and is configured to accommodate the at least one electrode contact and the signal processing chip. The catheter may be made of an elastic material, such as rubber, to be adapted to the curve and shape of biological tissue. Multiple electrode contacts are attached to an outer wall of the catheter. For example, 4 × 3 electrode contacts are provided. These electrode contacts are attached to the outer wall of the catheter and are configured to acquire the bioelectrical signals or deliver the electrical stimulation based on the stimulation control instruction. Each electrode contact may be in contact with different brain regions or neurons. The signal processing chip is located within the catheter and is configured to process the acquired signals and execute stimulation control. The signal processing chip is integrated with the signal parsing circuit and the signal acquisition circuit. The signal parsing circuit is configured to parse the stimulation control instruction from the pulse generator. For example, when the stimulator needs to deliver the electrical stimulation with a certain intensity at a specific time and location, the signal parsing circuit can decode these instructions into parameters of the stimulation pulse signal. The signal acquisition circuit is configured to convert the bioelectrical signals from the electrode contacts into digital signals. For example, if the electrode contacts record the electrical activity from a brain region, the signal acquisition circuit amplifies and filters these weak bioelectrical signals and converts them into the to-be-detected digital signals for subsequent processing and analysis.

Therefore, the integration of the signal processing chip into the electrode lead can reduce the external interference and the interference of the stimulation pulse signal on the bioelectrical signals. Conventionally, the signal parsing circuit and the signal acquisition circuit are integrated into the pulse generator, and the bioelectrical signals (analog signals) and the stimulation pulse signal are transmitted simultaneously through a cable. In the present application, the signal parsing circuit and the signal acquisition circuit that are originally located within the pulse generator are transplanted into the electrode lead in the form of an application-specific integrated circuit (ASIC). Using the signal processing chip, the bioelectrical signals (the analog signals) that were previously transmitted in the cable are converted into the digital signals. Compared with the analog signals, the digital signals possess stronger anti-interference capabilities. Furthermore, by disposing the signal processing chip within the lead, the bioelectrical signals can be processed closer to the electrode contacts, thereby reducing the impact of the external interference on the bioelectrical signals and further enhancing the anti-interference capabilities of the signals. In conclusion, the electrode lead provided in the present application can help improve the effectiveness and quality of acquiring the bioelectrical signals and reduce problems related to the external interference and the signal crosstalk interference.

The electrode lead provided in the present application can replace the analog signals transmitted in the cable with the digital signals, thereby mitigating the impact of the external environment (such as an electrocardiographic signal, an electrooculographic signal, an electromyographic signal, a strong external magnetic field, and a strong external electric field) and an internal strong electrical stimulation signal on the cable transmission. This can reduce the impact of a parsing deviation of the LFP signals on the closed-loop control of the IPG.

In some embodiments, the signal acquisition circuit includes an amplification module, a band-pass filtering module, and an analog-to-digital conversion module.

The amplification module is configured to amplify the bioelectrical signals to obtain first intermediate signals.

The band-pass filtering module is configured to perform band-pass filtering on the first intermediate signals to obtain second intermediate signals.

The analog-to-digital conversion module is configured to perform analog-to-digital conversion on the second intermediate signals to obtain the to-be-detected digital signals.

Therefore, the signal acquisition circuit is designed to include the amplification module, the band-pass filtering module, and the analog-to-digital conversion module so that the process of converting the bioelectrical signals into the to-be-detected digital signals encompasses amplification, filtering, and analog-to-digital conversion. The amplification module can increase the amplitude of each bioelectrical signal, making each bioelectrical signal easier to process and analyze. The band-pass filtering module can filter interference and noise from the external environment, thereby making a signal more reliable; filtering can also highlight the signal within a specific frequency range, which is useful for analyzing a particular physiological phenomenon. The analog-to-digital conversion module can convert the continuous analog signals into the digital signals, enabling the signals to be processed, stored, and analyzed on a digital device. These digital signals are easier to process and can be analyzed in a computer system. In conclusion, converting the bioelectrical signals into the to-be-detected digital signals can reduce errors that occur during the transmission and processing of the analog signals, thereby improving the accuracy and reliability of the signals.

In some embodiments, multiple groups of electrode contacts are arranged along a length direction of the catheter, and electrode contacts in each group are uniformly distributed in a circumferential direction of the catheter.

In some embodiments, for the electrode contacts in each group, when any electrode contact delivers the electrical stimulation to the internal tissue of the patient based on the stimulation pulse signal, part or all of other electrode contacts in the same group are used for acquiring bioelectrical signals of the patient.

In some embodiments, the electrode lead is provided with two to eight groups of electrode contacts. Each group includes one to nine electrode contacts.

The number of groups of electrode contacts is not limited in the embodiments of the present application. For example, three groups of electrode contacts, four groups of electrode contacts, five groups of electrode contacts, or eight groups of electrode contacts may be provided. The number of groups of electrode contacts may be set based on the size of the electrode lead and the size of the internal tissue. Correspondingly, the number of electrode contacts in each group is not limited. For example, each group may include two, three, four, six, or nine electrode contacts.

Therefore, in one aspect, the multiple electrode contacts can be divided into the multiple groups along the length direction of the catheter, and by providing segmented electrode contacts, the electrode contacts in each group can deliver independent and directional electrical stimulation, enabling the generated electrical stimulation to be targeted at a specific site or direction and thereby reducing overtreatment. In one aspect, multiple channels of bioelectrical signal acquisition and the electrical stimulation can be performed, and different combinations of electrode contacts can be used for performing the electrical stimulation and acquiring the bioelectrical signals respectively, which helps isolate electrical stimulation signals from the bioelectrical signals and reduces crosstalk interference. Uniformly distributing the multiple electrode contacts in the circumferential direction of the catheter can obtain a better spatial resolution, which helps locate and analyze sources of the bioelectrical signals. In another aspect, while one electrode contact delivers the electrical stimulation, other electrode contacts can be used for acquiring bioelectrical signals, thereby enabling simultaneous stimulation and signal acquisition. In this manner, the acquired bioelectrical signals can reflect the actual electroencephalographic activity of the patient during electrical stimulation treatment more accurately.

In some embodiments, the electrode lead is provided with four groups of electrode contacts, and each group includes three electrode contacts.

In the embodiments of the present application, the electrode lead is provided with a total of 4 × 3 electrode contacts, that is, 12 electrode contacts. Among the electrode contacts in each group, one electrode contact is used for the electrical stimulation, and the other two electrode contacts are used for acquiring LFP signals. Electrode contacts in a first group are denoted as 1a, 1b, and 1c sequentially; electrode contacts in a second group are denoted as 2a, 2b, and 2c sequentially; electrode contacts in a third group are denoted as 3a, 3b, and 3c sequentially; and electrode contacts in a fourth group are denoted as 4a, 4b, and 4c sequentially. 1a, 2a, 3a, and 4a are electrode contacts in the same column along an axial direction of a segmented electrode; 1b, 2b, 3b, and 4b are electrode contacts in the same column along the axial direction of the segmented electrode; and 1c, 2c, 3c, and 4c are electrode contacts in the same column along the axial direction of the segmented electrode.

Assuming that all the 12 electrode contacts are located in a nucleus, cyclic stimulation is performed using a fixed set of stimulation parameters (such as 4 V, 100 µs, and 150 Hz). That is, 1a delivers stimulation while 1b and 1c perform signal acquisition; 1b delivers stimulation while 1a and 1c perform signal acquisition; 1c delivers stimulation while 1a and 1b perform signal acquisition; 2a delivers stimulation while 2b and 2c perform signal acquisition; 2b delivers stimulation while 2a and 2c perform signal acquisition; 2c delivers stimulation while 2a and 2b perform signal acquisition; 3a delivers stimulation while 3b and 3c perform signal acquisition; 3b delivers stimulation while 3a and 3c perform signal acquisition; 3c delivers stimulation while 3a and 3b perform signal acquisition; 4a delivers stimulation while 4b and 4c perform signal acquisition; 4b delivers stimulation while 4a and 4c perform signal acquisition; and 4c delivers stimulation while 4a and 4b perform signal acquisition, which result in a total of 12 scenarios. For each scenario, a state in which acquisition is performed with no stimulation and a state in which stimulation and acquisition are performed simultaneously are each maintained for a preset duration. For example, the preset duration may be one minute, two minutes, or four minutes.

A bioelectrical signal acquired in the state in which acquisition is performed with no stimulation is denoted as a first electrical signal, and a bioelectrical signal acquired in the state in which stimulation and acquisition are performed simultaneously is denoted as a second electrical signal. The bioelectrical signals acquired by the electrode contacts include the first electrical signal and the second electrical signal.

Therefore, each group provides the three electrode contacts, resulting in a total of 12 electrode contacts available for use. This enables the electrode lead to simultaneously acquire bioelectrical signals from more channels, thereby providing richer and more comprehensive signal information. The three electrode contacts in each group can acquire signals at different angles and depths, which is highly useful for locating sources of the signals, analyzing signal changes, and studying the electrical activity characteristics of the internal tissue. By combining the electrode contacts in each group, multiple stimulation modes can be implemented, such as selectively stimulating different regions or adjusting stimulation intensity, which is highly valuable for some particular electrical stimulation treatment applications.

In some embodiments, the catheter is a rubber tube, the signal processing chip includes at least one bare die, the signal processing chip is electrically connected to each electrode contact via a metal wire, and the at least one bare die is electrically connected to each other via metal wires.

In some embodiments, the signal processing chip includes one to ten bare dies.

The number of bare dies is not limited in the embodiments of the present application. For example, one, two, three, four, seven, or ten bare dies may be provided.

When the three bare dies are used, the three bare dies can be assembled into a triangular prism structure; when the four bare dies are used, the four bare dies can be assembled into a rectangular cuboid structure.

A bare die refers to an exposed die without any packaging material or encapsulation. Compared with a conventionally packaged die, each bare die has a smaller size and a higher integration, occupying less space in an electronic device and providing faster data transmission and lower power consumption. The smaller size of each bare die shortens a signal transmission path, thereby reducing the probability of signal loss. Additionally, without any packaging material (such as plastic or ceramic), each bare die exhibits a better high-frequency response and a higher reliability.

In the embodiments of the present application, the metal wire may be a gold wire. The metal wires need to connect the electrode contacts on the outer wall of the catheter with the bare dies within the catheter. Therefore, the metal wires come into direct contact with the internal tissue of the patient (the nucleus). By using the gold wires, gold provides excellent electrical conductivity, ensuring signal transmission and enabling the bare dies to accurately read and transmit signals. Additionally, gold has strong oxidation resistance, helping maintain the long-term stability of the electrode lead. Furthermore, gold exhibits good biocompatibility, which reduces the rejection of the body of the patient against the electrode lead and prevents immune or inflammatory responses after the electrode lead is implanted into the brain, thereby improving safety and usability.

Therefore, using the rubber tube as the catheter provides a certain degree of flexibility, allowing the catheter to be adapted to the shapes of different organs and tissues. This flexibility helps improve the comfort and implantation experience of the patient. Using the metal wires to connect the signal processing chip to the electrode contacts can reduce signal transmission losses and interference. The metal wires can effectively transmit the bioelectrical signals and the stimulation pulse signal, helping maintain the integrity of the signals. Each electrode contact is electrically connected to the signal processing chip, and the bare dies are also connected to each other via the metal wires. This modular design of the chip makes subsequent maintenance, upgrades, and replacement much more convenient.

In some embodiments, the signal processing chip includes the three bare dies, and the three bare dies form the triangular prism structure.

In some embodiments, an adhesive is filled between the signal processing chip and an inner wall of the catheter to secure the signal processing chip within the catheter.

The adhesive is not limited in the embodiments of the present application and may be glue.

Therefore, the triangular prism structure can effectively utilize the internal space of the catheter, allowing the signal processing chip to maintain a more stable structure within the limited space. This compact structure also helps simplify the layout of the electrode lead. The adhesive is used for securing the signal processing chip within the catheter so that the signal processing chip can be prevented from being loosened or displaced during use, thereby enhancing the stability and reliability of the electrode lead. In one aspect, directly securing the signal processing chip within the catheter can optimize the signal transmission path, reducing the signal transmission losses and interference and thus improving the quality of the signals; in another aspect, completely packaging the signal processing chip within the catheter can reduce the impact of the external environment on the signal processing chip, further enhancing the stability and accuracy of the signals.

In some embodiments, the internal tissue of the patient includes subthalamic nucleus, and the bioelectrical signals include the local field potential signals.

Therefore, as an important region of the brain, the subthalamic nucleus involves various neural functions, such as motor control and emotional adjustment. By acquiring the local field potential signals, detailed information about the activity of the subthalamic nucleus can be acquired, which is helpful for studying the function and modulation mechanism of the subthalamic nucleus. In clinical applications, treatment for a certain patient (such as a patient with Parkinson's disease) may need to be adjusted based on the activity of the subthalamic nucleus of the patient. Acquiring the local field potential signals can provide essential physiological information for personalized treatment. Moreover, directional electrical stimulation can be delivered to the subthalamic nucleus through the stimulation pulse signal, which plays a significant role in neuromodulation treatment for the patient with Parkinson's disease.

In a specific application scenario, the present application provides the electrode lead. The electrode lead includes the catheter, the at least one electrode contact, and the signal processing chip.

Each electrode contact is attached to the outer wall of the catheter, and each electrode contact is configured to perform at least one of the following: acquiring the bioelectrical signal of the patient, or delivering the electrical stimulation to the internal tissue of the patient based on the stimulation pulse signal. The internal tissue of the patient includes the subthalamic nucleus, and the bioelectrical signal includes the local field potential signal.

The signal processing chip is disposed within the catheter and is electrically connected to the at least one electrode contact. The signal processing chip is integrated with the signal parsing circuit and the signal acquisition circuit, where the signal parsing circuit is configured to parse the stimulation control instruction sent by the pulse generator and convert the stimulation control instruction into the stimulation pulse signal, and the signal acquisition circuit is configured to convert bioelectrical signals acquired by the at least one electrode contact into to-be-detected digital signals and output the to-be-detected digital signals.

The four groups of electrode contacts are arranged along the length direction of the catheter, the electrode contacts in each group are uniformly distributed in the circumferential direction of the catheter, and each group includes the three electrode contacts.

For the electrode contacts in each group, when the any electrode contact delivers the electrical stimulation to the internal tissue of the patient based on the stimulation pulse signal, the part or all of the other electrode contacts in the same group are used for acquiring the bioelectrical signals of the patient.

The catheter is the rubber tube, the signal processing chip includes the three bare dies that form the triangular prism structure, the signal processing chip is electrically connected to each electrode contact via the metal wire, and the three bare dies are electrically connected to each other via the metal wires.

The adhesive is filled between the signal processing chip and the inner wall of the catheter to secure the signal processing chip within the catheter.

The signal acquisition circuit includes the amplification module, the band-pass filtering module, and the analog-to-digital conversion module. The process of converting the bioelectrical signals into the to-be-detected digital signals includes the following steps.

The amplification module is used for amplifying the bioelectrical signals to obtain the first intermediate signals.

The band-pass filtering module is used for performing the band-pass filtering on the first intermediate signals to obtain the second intermediate signals.

The analog-to-digital conversion module is used for performing the analog-to-digital conversion on the second intermediate signals to obtain the to-be-detected digital signals.

In an embodiment, a softer and more comfortable material is introduced into the design of the catheter to be better adapted to the physiological structure of the patient, thereby enhancing wearing comfort and reducing interference with biological tissue. For the signal processing chip, the signal parsing circuit and the signal acquisition circuit can be optimized to achieve higher signal enhancement and noise reduction, thereby improving the quality and accuracy of the signals. For example, the multiple stimulation modes can be added so that the stimulator can operate at different frequencies, amplitudes, and durations, thereby better satisfying the requirements of different diseases or therapeutic objectives. A real-time feedback mechanism is introduced, in which the acquired bioelectrical signals are converted into visual or auditory feedback, helping the patient or the doctor better understand the physiological state and stimulation effects of the patient. A processor of the pulse generator is optimized to adaptively adjust the stimulation parameters based on the bioelectrical signals acquired in real time, thereby providing more precise stimulation. Remote monitoring and control functions are introduced to allow the doctor or a researcher to remotely adjust the stimulation parameters, observe the quality of the signals, and perform real-time data analysis. Wireless communication technologies, such as Bluetooth or Wi-Fi, are employed to enable a wireless connection between the stimulator/a stimulator system and an external device, reducing restrictions caused by the wire. Regarding the selection of materials for the wire and a housing, optimization is performed to ensure long-term stability and biocompatibility. In this manner, adequate data security measures are used throughout the processes of signal acquisition, transmission, and storage, so as to protect the privacy of the patient.

### Embodiment of the stimulator system

Referring to FIG. 3, FIG. 3 is a diagram illustrating the principle of signal transmission of a stimulator system according to an embodiment of the present application.

The embodiment of the present application further provides the stimulator system. The stimulator system includes a pulse generator and the electrode lead according to any one of the preceding solutions, and the pulse generator is electrically connected to the signal processing chip in the electrode lead.

In some embodiments, the pulse generator includes a memory and at least one processor, the memory stores a computer program, and when executing the computer program, the at least one processor is configured to perform the following steps.

To-be-detected digital signals output by the signal processing chip are received.

A corresponding stimulation control instruction is generated according to the to-be-detected digital signals, and the stimulation control instruction is output to the signal processing chip.

In some embodiments, the stimulator system further includes an extension lead. The pulse generator may be electrically connected to the signal processing chip via the extension lead (as shown in FIG. 1), or may also be directly electrically connected to the signal processing chip (as shown in FIG. 2).

In some embodiments, when executing the computer program, the at least one processor is further configured to perform the following steps.

Disease information of the patient is acquired.

A first duration and a second duration within a signal acquisition period are set according to the disease information and the to-be-detected digital signals, where multiple electrode contacts are provided.

During the first duration, at least one electrode contact is used for acquiring bioelectrical signals of the patient while the electrical stimulation is delivered to the internal tissue of the patient.

During the second duration, at least one electrode contact is used for acquiring bioelectrical signals of the patient while no electrical stimulation is delivered.

For example, during the first duration, the at least one electrode contact transmits the stimulation pulse signal to the internal tissue of the object, and at least one of the remaining electrode contacts acquires the bioelectrical signals of the object.

During the second duration, the at least one electrode contact does not transmit the stimulation pulse signal, and at least one of the remaining electrode contacts acquires the bioelectrical signals of the object. In an embodiment, the first duration and the second duration within the signal acquisition period may be set by using a pre-trained deep learning model according to the disease information and the to-be-detected digital signals.

In the embodiment of the present application, the first duration and the second duration jointly form one signal acquisition period. The first duration and the second duration may be equal or may be different from each other. For example, the first duration may be one minute, two minutes, or four minutes, and the second duration may be one minute, two minutes, or four minutes.

As an example, the first duration is one minute, and the second duration is one minute.

Assuming that all the 12 electrode contacts are located within the nucleus, the electrode contacts in the first group are denoted as 1a, 1b, and 1c. During the first minute, 1a delivers stimulation while 1b and 1c perform signal acquisition, and during the second minute, 1a stops delivering the stimulation, and 1b and 1c perform the signal acquisition. Subsequently, during the third minute, 1b delivers stimulation while 1a and 1c perform signal acquisition, and during the fourth minute, 1b stops delivering the stimulation while 1a and 1c perform the signal acquisition. Afterwards, during the fifth minute, 1c delivers stimulation while 1a and 1b perform signal acquisition, and during the sixth minute, 1c stops delivering the stimulation while 1a and 1b perform the signal acquisition. In this manner, this process continues until all the groups of electrode contacts have performed both acquisition modes: simultaneous stimulation and acquisition within each group and acquisition-only without stimulation within each group.

Therefore, the first duration and the second duration within the signal acquisition period are set according to the disease information of the patient and the to-be-detected digital signals. During the first duration, through the electrode contacts, the stimulator system simultaneously delivers the electrical stimulation and acquires the bioelectrical signals (simultaneous acquisition and stimulation). During the second duration, the stimulator system acquires the bioelectrical signals without delivering the electrical stimulation (acquisition-only without stimulation). In this manner, the bioelectrical signals of the patient under the electrical stimulation and the bioelectrical signals of the patient without the electrical stimulation can be obtained. Jointly analyzing and comparing these two types of bioelectrical signals can provide an accurate basis for closed-loop stimulation.

In a specific application scenario, the present application provides the stimulator system. The stimulator system includes the pulse generator and the electrode lead, and the pulse generator is electrically connected to the signal processing chip in the electrode lead via the extension lead.

The pulse generator includes the memory and the at least one processor, the memory stores the computer program, and when executing the computer program, the at least one processor is configured to perform the following steps.

The to-be-detected digital signals output by the signal processing chip are received.

The corresponding stimulation control instruction is generated according to the to-be-detected digital signals, and the stimulation control instruction is output to the signal processing chip.

The disease information of the patient is acquired.

The first duration and the second duration within the signal acquisition period are set according to the disease information and the to-be-detected digital signals, where multiple electrode contacts are provided.

During the first duration, the at least one electrode contact is used for acquiring the bioelectrical signals of the patient while the electrical stimulation is delivered to the internal tissue of the patient.

During the second duration, at least one electrode contact is used for acquiring the bioelectrical signals of the patient while no electrical stimulation is being delivered.

In an embodiment, multiple sensors are added to the electrode lead to capture more bioelectrical signals, such as electrocardiographic signals, electrooculographic signals, and electromyographic signals, thereby providing more comprehensive information for treatment. An adaptive energy-management system is integrated into the stimulator system to intelligently allocate energy according to real-time energy demands, thereby improving battery life or the efficiency of wireless charging. Appropriate privacy-protection and security measures are taken to safeguard biological data of the patient, while both the patient and the doctor are allowed to control data sharing and storage. A cloud-based platform is used for enabling remote monitoring and data transmission for the stimulator system so that the doctor can remotely adjust the stimulation parameters and monitor the state of the patient in real time. Customized therapeutic solutions are developed for different types of diseases and patients to ensure that the stimulator system performs optimally across diverse conditions. Close collaboration with a neuroscientist, a clinician, and an engineer is undertaken to ensure that the design and application of the stimulator system are feasible and advantageous across scientific, medical, and engineering fields. Long-term stability testing is conducted to ensure that the stimulator system remains stable and reliable during extended use.

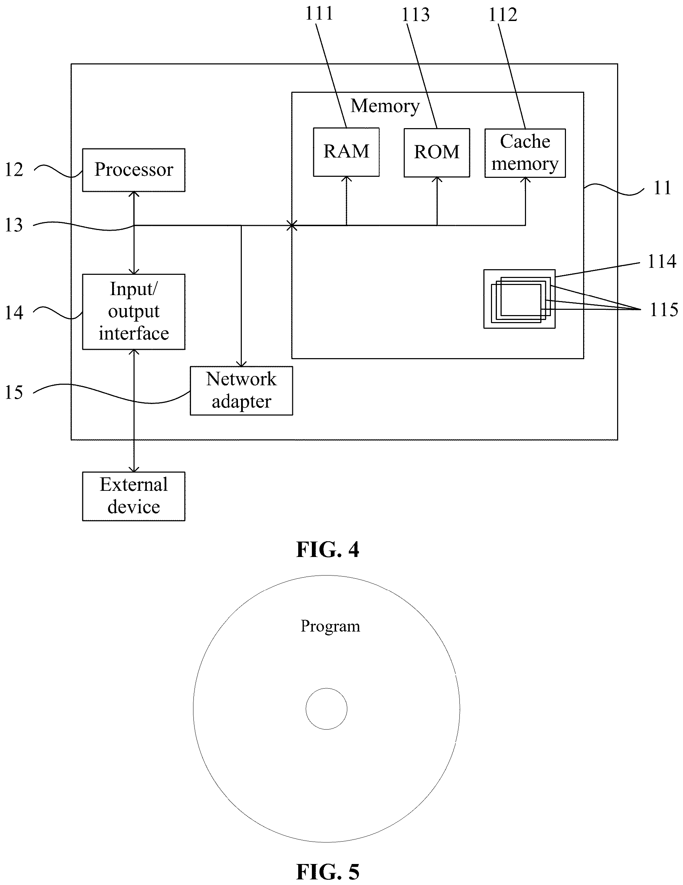

Referring to FIG. 4, FIG. 4 is a block diagram of a stimulator system according to an embodiment of the present application.

For example, the stimulator system may include at least one memory 11, at least one processor 12, and a bus 13 connecting different platform systems.

The memory 11 may include a readable medium in the form of volatile memory, such as a random-access memory (RAM) 111 and/or a cache memory 112, or may further include a read-only memory (ROM) 113.

The memory 11 further stores a computer program that, when executed by the processor 12, causes the processor 12 to perform the preceding steps.

The memory 11 may further include a utility 114 having at least one program module 115. Such a program module 115 includes, but is not limited to, an operating system, one or more application programs, other program modules, and program data. Each or some combination of these examples may include implementation of a network environment.

Correspondingly, the processor 12 can execute the preceding computer program and can also execute the utility 114.

The processor 12 may be implemented using one or more application-specific integrated circuits (ASICs), digital signal processors (DSPs), programmable logic devices (PLDs), complex programmable logic devices (CPLDs), field-programmable gate arrays (FPGAs), or other electronic elements.

The bus 13 may represent one or more of several types of bus architectures, including a memory bus or memory controller, a peripheral bus, an Accelerated Graphics Port, and a processor, or may represent a local bus using any one of multiple bus architectures.

The stimulator system may also communicate with one or more external devices such as a keyboard, a pointing device, and a Bluetooth device. The stimulator system may also communicate with one or more devices that can interact with the stimulator system, and/or with any device (such as a router or a modem) that enables the stimulator system to communicate with one or more other computing devices. Such communications may be performed through an input/output interface 14. Moreover, the stimulator system may also communicate with one or more networks (such as a local area network (LAN), a wide area network (WAN), and/or a public network such as the Internet) through a network adapter 15. The network adapter 15 can communicate with other modules of the stimulator system via the bus 13. Although not shown in the figure, other hardware and/or software modules may be used in conjunction with the stimulator system in practical applications. The other hardware and/or software modules include, but are not limited to, microcode, a device driver, a redundant processor, an external disk drive array, a redundant arrays of independent disks (RAID) system, a tape driver, and a data backup storage platform.

### Embodiment of the computer-readable storage medium

An embodiment of the present application further provides a computer-readable storage medium. Embodiments of the computer-readable storage medium achieve the same technical effects as the preceding embodiments, and details are not repeated herein.

The computer-readable storage medium stores a computer program that, when executed by at least one processor, causes the at least one processor to perform the preceding steps or fulfill functions of the stimulator according to any one of the preceding solutions.

The computer-readable medium may be a computer-readable signal medium or a computer-readable storage medium. In the embodiment of the present application, the computer-readable storage medium may be any tangible medium including or storing a program. The program may be used by or in conjunction with an instruction execution system, apparatus, or device. For example, the computer-readable storage media may be, but are not limited to, an electrical, magnetic, optical, electromagnetic, infrared or semiconductor system, apparatus or device, or any combination thereof. The computer-readable storage medium may include (non-exhaustive list): an electrical connection having one or more wires, a portable computer magnetic disk, a hard disk, a random-access memory (RAM), a read-only memory (ROM), an erasable programmable read-only memory (EPROM) or a flash memory, an optical fiber, a portable compact disk read-only memory (CD-ROM), an optical storage device, a magnetic storage device, or any suitable combination thereof.

The computer-readable storage medium may include a data signal propagated on a base band or as a part of a carrier wave. The data signal carries readable program codes. The data signal propagated in this manner may be in multiple forms and includes, but is not limited to, an electromagnetic signal, an optical signal, or any suitable combination thereof. The computer-readable storage medium may also be any computer-readable medium that can send, propagate, or transmit a program used by or in conjunction with an instruction execution system, apparatus, or device. The program codes included on the computer-readable storage medium may be transmitted by using any suitable medium, including, but not limited to, a wireless medium, a wired medium, an optical cable, radio frequency (RF), and the like, or any suitable combination thereof. Program codes for performing the operations of the present application may be written in one or more programming languages or a combination thereof, the programming languages including object-oriented programming languages such as Java and C++, and further including conventional procedural programming languages such as C programming language or similar programming languages. The program codes may be executed entirely on a user computing device, executed partly on a user device, executed as a stand-alone software package, executed partly on a user computing device and partly on a remote computing device, or executed entirely on a remote computing device or a server. In the case where a remote computing device is involved, the remote computing device may be connected to a user computing device via any type of network including a local area network (LAN) or a wide area network (WAN), or may be connected to an external computing device (for example, via the Internet provided by an Internet service provider).

### Embodiment of a computer program product

An embodiment of the present application further provides a computer program product. Embodiments of the computer program product achieve the same technical effects as the preceding embodiments, and details are not repeated herein.

The present application provides the computer program product including a computer program that, when executed by at least one processor, causes the at least one processor to perform the preceding steps or fulfill functions of the stimulator according to any one of the preceding solutions.

Referring to FIG. 5, FIG. 5 is a diagram illustrating the structure of a computer program product according to an embodiment of the present application.

The computer program product is configured to perform the steps of the method according to any one of the preceding solutions or fulfill the functions of the stimulator according to any one of the preceding solutions. The computer program product may be implemented using a portable compact disk read-only memory (CD-ROM), include program codes, and run on a terminal device such as a personal computer. However, the computer program product of the present application is not limited to this and may be implemented using any combination of one or more computer-readable media.

## Claims

1. An electrode lead, comprising a catheter, at least one electrode contact, and a signal processing chip,
wherein each electrode contact of the at least one electrode contact is attached to an outer wall of the catheter, and each electrode contact is configured to perform at least one of the following:
acquiring a bioelectrical signal of an object, or delivering electrical stimulation to internal tissue of the object based on a stimulation pulse signal; and
wherein the signal processing chip is disposed within the catheter and is electrically connected to the at least one electrode contact.

2. The electrode lead according to claim 1, wherein the signal processing chip is integrated with a signal parsing circuit and a signal acquisition circuit, wherein the signal parsing circuit is configured to parse a stimulation control instruction sent by a pulse generator and convert the stimulation control instruction into the stimulation pulse signal, and the signal acquisition circuit is configured to convert bioelectrical signals acquired by the at least one electrode contact into to-be-detected digital signals and output the to-be-detected digital signals.

3. The electrode lead according to claim 1, wherein a plurality of groups of electrode contacts are arranged along a length direction of the catheter, and electrode contacts in each group of the plurality of groups of electrode contacts are uniformly distributed in a circumferential direction of the catheter.

4. The electrode lead according to claim 3, wherein for the electrode contacts in each group, when any electrode contact delivers the electrical stimulation to the internal tissue of the object based on the stimulation pulse signal, part or all of other electrode contacts in a same group acquire bioelectrical signals of the object.

5. The electrode lead according to claim 3, wherein the electrode lead is provided with two to eight groups of electrode contacts.

6. The electrode lead according to claim 5, wherein the electrode lead is provided with four groups of electrode contacts.

7. The electrode lead according to claim 3, wherein each group comprises one to nine electrode contacts.

8. The electrode lead according to claim 7, wherein each group comprises three electrode contacts.

9. The electrode lead according to claim 1 or 2, wherein the catheter is a rubber tube, the signal processing chip comprises at least one bare die, the signal processing chip is electrically connected to each electrode contact via a metal wire, and the at least one bare die is electrically connected to each other via metal wires.

10. The electrode lead according to claim 9, wherein the signal processing chip comprises one to ten bare dies.

11. The electrode lead according to claim 10, wherein the signal processing chip comprises three bare dies.

12. The electrode lead according to claim 11, wherein the three bare dies form a triangular prism structure.

13. The electrode lead according to claim 9, wherein an adhesive is filled between the signal processing chip and an inner wall of the catheter to secure the signal processing chip within the catheter.

14. The electrode lead according to claim 2, wherein the signal acquisition circuit comprises an amplification module, a band-pass filtering module, and an analog-to-digital conversion module that are connected sequentially;
the amplification module is configured to amplify the bioelectrical signals to obtain first intermediate signals;
the band-pass filtering module is configured to perform band-pass filtering on the first intermediate signals to obtain second intermediate signals; and
the analog-to-digital conversion module is configured to perform analog-to-digital conversion on the second intermediate signals to obtain the to-be-detected digital signals.

15. The electrode lead according to claim 1 or 2, wherein the internal tissue of the object comprises subthalamic nucleus.

16. The electrode lead according to claim 1 or 2, wherein the bioelectrical signal comprises a local field potential signal.

17. A stimulator system, comprising a pulse generator and the electrode lead according to any one of claims 1 to 16, wherein the pulse generator is electrically connected to the signal processing chip in the electrode lead.

18. The stimulator system according to claim 17, wherein the pulse generator comprises a memory and at least one processor, the memory stores a computer program, and when executing the computer program, the at least one processor is configured to perform the following steps:
receiving to-be-detected digital signals output by the signal processing chip; and
generating a stimulation control instruction according to the to-be-detected digital signals and outputting the stimulation control instruction to the signal processing chip.

19. The stimulator system according to claim 18, wherein when executing the computer program, the at least one processor is further configured to perform the following steps:
acquiring disease information of the object; and
setting a first duration and a second duration within a signal acquisition period according to the disease information and the to-be-detected digital signals, wherein multiple electrode contacts are provided,
wherein during the first duration, at least one electrode contact of the electrode contacts transmits the stimulation pulse signal to the internal tissue of the object, and at least one of remaining electrode contacts of the electrode contacts acquires bioelectrical signals of the object; and
wherein during the second duration, the at least one electrode contact of the electrode contacts does not transmit the stimulation pulse signal, and at least one of the remaining electrode contacts of the electrode contacts acquires bioelectrical signals of the object.

20. A computer-readable storage medium storing a computer program that, when executed by at least one processor, is configured to fulfill functions of the stimulator system according to any one of claims 17 to 19.
